# EUROPEAN PATENT APPLICATION

(11) **EP 2 065 870 A1**
(43) Date of publication of application: **03.06.2009**
(21) Application number: 07022538.8
(22) Date of filing: 21.11.2007
(51) Int. Cl.: G09B 21/00, A61B 5/00, G06F 3/01

(54) **Medical device for visually impaired users and users not visually impaired**

(71) Applicant: Roche Diagnostics GmbH, 68298 Mannheim (DE); F.HOFFMANN-LA ROCHE AG, 4070 Basel (CH)
(72) Inventor: Eisenhardt, Christoph, 68165 Mannheim (DE); Voelkel, Dirk, 69469 Weinheim (DE)
(74) Representative: Jung, Michael

(57) **Abstract**

The present invention refers to a medical device for self testing and monitoring the concentration of analytes in a body fluid, or a blood parameter, or for the controlled administration of therapeutic agents, comprising a connectable, preferably pluggable Braille or Braille-like module for visually impaired users, so that both visually impaired users and users not visually impaired can read the data generated by the medical device and/or control the medical device.

## Description

### Field of the invention

The present invention refers to a medical device, in particular for self testing and monitoring the concentration of analytes in a body fluid, or a blood parameter, or for the controlled administration of therapeutic agents, comprising a connectable, preferably pluggable Braille or Braille-like module for visually impaired users, so that both visually impaired users and users not visually impaired can read the data generated by the medical device and/or control the medical device.

### Background

In many fields of healthcare, repeated measurement and monitoring of certain analytes present in body fluids, such as blood or urine, is of particular importance. One special case concerns for example patients affected by diabetes who need to measure very frequently the concentration of glucose in order to respond promptly with the correct medication. If certain limits are indeed exceeded, the affected patient might even incur in coma and death. The high concentration of glucose in the blood is also responsible for well known side effects gradually deteriorating the state of health. Therefore long term monitoring is required in order to keep the glycemic level under control. Blood glucose data are thus useful both to the physician who has the task to determine the most appropriate long-term therapy and to the patient who daily needs to adapt the administration of medications according to the measured glucose levels. These depend in fact not only on the diet, but also on the daily physical activity and many other factors, which influence the metabolism.

A number of small, reliable and low-cost medical devices, which can be handheld, are today available to the patient for self monitoring. Devices for controlled administration of therapeutic agents, such as insulin pumps, are also commercially available.

The number of examples of medical devices to which this invention refers to is however not limited to diabetes care. Worth mentioning are for example also those devices for monitoring blood pressure or other blood parameters like the coagulation factors.

A complication associated with the use of such medical devices arises when the patient or user is also visually impaired. This health condition might exist for example since birth or following a trauma, but progressive visual impairment eventually culminating in blindness is also one of the long-term side effects, cited above, caused by the high concentration of glucose in blood. In other words, it is relatively frequent for diabetic patients to be also visually impaired. Thus it is particularly by these that a valid solution to this technical problem is highly desired.

There are known examples of medical devices on the market such as glucose meters or insulin pumps which partly solve this problem by providing a talking function, i. e. providing an audible output. This function can be either directly integrated into the medical device with considerable development costs for each different medical device or indirectly provided through a second device, e. g. in wireless communication with the medical device like the Accu-Chek^{®} Voicemate device by Roche Diagnostics, described in EP1728470. The main advantage in the latter case is that a single talking device can be used for several medical devices, thus partly reducing the development costs, although these remain high.

A disadvantage of any acoustic approach remains however the fact that discretion and privacy are compromised any time the user is not alone, either at home or in a public place. This function may be therefore not desired by the user in every situation.

Another approach would be that based on tact. For example, the Braille code, devised in 1821 by Louis Braille, is widely used by blind people to read and write. Each Braille character is made up of six dots, which can be raised e.g. by relief printing or Braille embosser typically on paper, and as such sensed by the fingertip upon contact. These six dots are arranged in a rectangle or cell containing two columns of three dots each. A dot may be raised at any of the six positions to form sixty-four (2⁶) possible combinations.

Now, while static Braille or contour text can be relatively easily achieved on paper or plastic sheets, far more complicated is to integrate an electro-mechanical Braille display on a medical device. Such displays, so called refreshable Braille displays or Braille terminals, provided especially as interface for computers, exist but are extremely expensive, compared to the medical device itself. Hence, this approach becomes technically and commercially unsuitable.

The theoretical possible use of Braille displays for medical devices such as glucose meters was mentioned already in documents like WO 2005/065539, WO 2005/086744 and WO 2006/026741. These failed however to specify what sort of Braille display could have been used and how it would have been used.

In general, the development of a dedicated medical device, which is suitable just for visually impaired users remains economically inconvenient.

It is therefore an object of the present invention to reduce the development costs of a medical device for visually impaired users. This is achieved by the present invention by means of a tactile display module for visually impaired users connectable, preferably pluggable into a medical device, wherein the medical device is the same developed for users not visually impaired. At the same time the development costs of said module are also low thanks to a new emerging technology making use of thin and flexible plastic sheets, organic transistors and plastic actuators, particularly suitable for Braille and Braille-like displays. This technology is described in more detail in Kato, Y. et al, IEEE Transactions on Electron Devices, 2007, 54, 202-208.

A further advantage of the present invention is to provide a medical device, which is not merely intended for visually impaired users, but for both visually impaired users and users not visually impaired, allowing information exchange between them. For example, it could happen that a visually impaired user needs to show the result to another person or needs once assistance, when using the medical device, by another person, e.g. a family member or a doctor.

This advantage is achieved by the present invention in that two types of display, one for each user category, that is a visual display for users not visually impaired and a tactile display module for visually impaired users, can be used together with only one medical device and preferably even can be used simultaneously.

By means of this invention the disadvantage of the acoustic function as well as the need of another expensive device in communication with the medical device is also avoided.

### Description of the invention

The present invention refers to a system comprising a medical device and a tactile display module, wherein the medical device comprises an analytical test section or a drug administration section or both;
a visual display for users not visually impaired displaying data generated by the medical device and/or instructions for operating the medical device; wherein the tactile display module is connected to and preferably is plugged into the medical device; and wherein the tactile display module can transmit data generated by the medical device and/or instructions for operating the medical device to visually impaired users.

A medical device according to the present invention is preferably a handheld device for self testing and monitoring the concentration of analytes in a body fluid such as blood or urine, or a blood parameter such as blood pressure or blood coagulation factors. Examples are glucose meters, coagulation factor meters, blood pressure meters. A medical device may however concern also the controlled administration of therapeutic agents. Examples are insulin pumps. A medical device may not only report current and/or memorized data generated during its use, e.g. the concentration value of a measured analyte, but display other information, too, such as the time of the measurement, a batch number of the test strips used, the dose and time of a therapeutic agent delivered, etc. as well as give operational instructions during its use.

A medical device may comprise either an analytical test section or a drug administration section or both. An analytical test section is a part of the medical device by means of which the blood parameter, in particular the concentration of one or more analytes in a body fluids can be measured and the information obtained used for diagnostic purposes. An analytical test section may comprise for example a port to accommodate an analytical test strip and an optical or electrochemical detector. Disposable lancets operated by the medical device may be also part of the analytical test section. Measurement steps may involve also calibration steps and/or reading of batch numbers. A suitable medical device is e.g. described in US 6379317 B1. The medical device additionally or alternatively may comprise a drug administration section. This section - may comprise for example a pump, e.g. a syringe pump, a syringe, a capsule or vial, filled with the drug to be administrated, an infusion catheter. A suitable medical device comprising a drug administration section is e.g. described in US 2004/0006310 A1. An example of drug is insulin and its administration over time is controlled by the medical device either as planned infusion doses or dynamically in response to the measured concentration of glucose in a body fluid.

Such concentration of glucose may be measured by means of the analytical test section of the same or a different medical device.

According to the present invention a visual display is a display integrated into the medical device, displaying numbers and/or letters and/or symbols recognizable by the sense of sight by a person with normal or sufficient visual capability. A suitable visual display is for example a standard LCD (Liquid Crystal Display) or an OLED display. Another suitable example is an electronic paper (e-paper) display based on e-ink technology. Other types of displays are available and are well known to those skilled in the art and are principally also suitable for the present invention.

According to the present invention the tactile display module is a display, that is connectable to and in particular pluggable into the medical device, displaying signs, i.e. numbers and/or letters and/or symbols recognizable by the sense of tact. These signs could be conventional signs taken from a conventional written language or graphic signs displayed as contours or relief (hereinafter referred to as "Braille-like displays"). Alternatively, a special code developed for visually impaired persons could be employed. An example of such a code or language is the Braille code. The tactile display module according to the present invention is preferably a Braille module or a Braille-like module comprising a thin sheet display based on the Braille code or on conventional language signs, wherein the sheet display comprises preferably organic field-effect transistors and plastic actuators.

Altogether, the thin sheet display consists preferably of at least three main layers: an organic field-effect transistor thin film deposited on a plastic sheet, for short a transistor layer, a thin plastic or polymeric actuator layer on top of the transistor layer, and a thin polymeric cover layer on top of all, with the feature of being elastically deformable.

An organic field-effect transistor is a class of thin film transistors based on organic semiconductors. A thin film transistor is made by depositing thin films of metallic contacts, a semiconductor active layer, in this case an organic semiconductor, and a dielectric layer onto a substrate, in this case a plastic film. An organic semiconductor is any organic material that has semiconductor properties. A semiconductor is any compound whose electrical conductivity is between that of typical metals and that of insulating compounds. Both short chain (oligomers) and long chain (polymers) organic semiconductors are known. Examples of semiconducting oligomers are: pentacene, anthracene and rubrene. Examples of polymers are: poly(3-hexylthiophene), poly(p-phenylene vinylene), F8BT, as well as polyaectylene and its derivatives.

The plastic actuators making the thin actuator layer consist preferably of an ionic polymer metal composite or any other of the several available soft dielectric elastomers or conjugated polymers. An introduction to these materials is given by Shahinpoor, M. et al, Smart Mater. Struct., 1998, 7, R15-R17.

According to the present invention the medical device further comprises a display interface and the tactile display module is connected to the medical device via said interface or bus.

In computer architecture, an interface or bus is a subsystem that transfers data and/or power via wire, i.e. via conductors, between computer components inside a computer or between a computer and a peripheral device.

Some interfaces may be preferred to attach low-speed peripherals to a motherboard, an embedded system such as a medical device, or a cellphone. In this case the peripheral is the tactile display module and the display interface allows data and preferably also power transfer between the medical device and the tactile display module. This transfer preferably is achieved via a wire connection between the display module and the medical device. It is, however, also possible that data are exchanged in a wireless mode, e. g. via infrared or radio frequency transmission. Examples of suitable display interfaces according to the present inventions are: Serial Peripheral Interface Bus (SPI bus), Universal Serial Bus (USB), Inter-Integrated Circuit (I²C), Enhanced Graphics Adapter (EGA), Video Graphics Array (VGA), Digital Visual Interface (DVI), Unified Display Interface (UDI). Other suitable interfaces are well known to those skilled in the art and are principally also suited for the purposes of the present invention.

In a preferred embodiment, the object of the present invention is thus achieved by means of Braille or Braille-like module preferably made with organic field-effect transistors and plastic actuators pluggable into said display interface of the medical device, wherein the medical device is the same developed for users not visually impaired comprising a visual display. In other words, the present invention discloses a medical device, which is preferably developed for both visually impaired and for not visually impaired users. Indeed, the medical device comprises preferably an analytical test section or a drug administration section or both; a visual display for users not visually impaired displaying data generated by the medical device and/or giving instructions for operating the medical device; and a display interface into which a tactile display module for visually impaired users transmitting data generated by the medical device and/or giving instructions for operating the medical device can be plugged.

When the user needing the medical device is visually impaired the tactile display module for visually impaired users is plugged into the medical device so that both visually impaired users and users not visually impaired can read the data generated or receive instructions given by the medical device, and/or control the medical device.

The tactile display module is preferably so manufactured that the thin sheet display is comprised in a sort of solid frame or body and assumes at least partly a rigid shape.

Upon plugging, the tactile display module can be disposed in different ways relative to the medical device. For example use of a relatively long and flexible cable could be made and the module may lay at one side of the medical device. Preferably, however, for size optimization reasons, the body of the tactile display module is firmly fixed, at least at one point, to the medical device and preferably can be disposed over the visual display. Particularly, the module can be made to rotate along an axis or slide along guide lines in order to expose the visual display whenever desired. Firm fixing to the medical device could be accomplished at the display interface itself if the module body is suitably designed, e.g. equipped with a rigid plug, which is continuous part of the rigid body, while for example being still capable of rotating at this point. Otherwise, the module could be plugged via a relatively short and flexible wiring and firm fixing of the module body to the medical device occurs at a different point.

More in detail, the present invention can be best understood when read in conjunction with the following drawings, representing favorite embodiments, in which:
Figure 1 represents schematically a medical device in use by a person not visually impaired.
Figure 2 represents schematically a system comprising the same medical device as in figure 1 and a plugged in tactile display module in use by a person visually impaired.

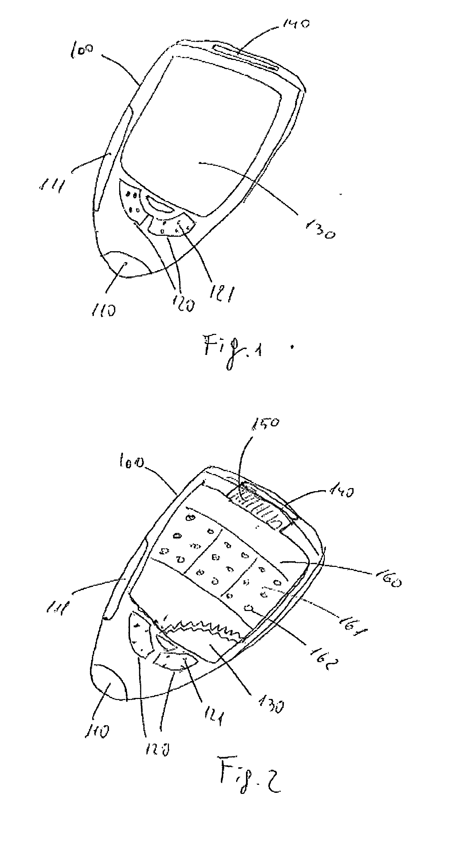

In figure 1 a medical device 100 in use by a person not visually impaired is shown. According to one embodiment the medical device 100 comprises preferably an analytical test section 110. According to another embodiment the medical device 100 comprises preferably a drug administration section 111. According to another embodiment the medical device 100 comprises both an analytical test section 110 and a drug administration section 111.

The medical device 100 comprises preferably also functional buttons 120 to control the operation of the medical device 100. These buttons 120 might comprise visual signs or written text such as "on/off" and preferably also signs 121, by which the same function is interpretable by tact, e.g. Braille characters.

The medical device 100 comprises preferably a standard visual display 130, e.g. an LCD or an e-paper display, to display operational instructions during use and/or data generated or memorized by the medical device 100.

According to the present invention the medical device 100 comprises also a display interface 140, into which a module 160 for visually impaired users can be plugged, wherein the display interface 140 is preferably any of the following: a Serial Peripheral Interface Bus (SPI bus), a Universal Serial Bus (USB), an Inter-Integrated Circuit (I²C), an Enhanced Graphics Adapter (EGA), a Video Graphics Array (VGA), a Digital Visual Interface (DVI), a Unified Display Interface (UDI).

In figure 2 a system is shown comprising the same medical device 100 of figure 1 and a tactile display module 160 for visually impaired users plugged into the display interface 140 of the medical device 100 by means of wiring 150. Wiring 150 apart from allowing data transfer between the medical device 100 and the tactile display module 160 preferably provides also power to the tactile display module 160.

The tactile display module 160 is preferably a Braille module comprising Braille cells 161, each cell comprising six dot positions 162, which can be raised or remain flat depending on the character to display. To each cell 161 corresponds thus a character or symbol to be interpreted by tact, e.g. by sliding a fingertip, by a visually impaired user.

Alternatively, the tactile display module 160 is a Braille-like module comprising Braille-like cells 161, each cell comprising an array of dot positions 162, which can be raised or remain flat depending on the conventional language sign to display. In this case, to each cell 161 corresponds thus a conventional character, e.g. letter, number or symbol to be interpreted by tact, e.g. by sliding a fingertip, by a visually impaired user.

The tactile display module 160 is preferably disposed, for size optimization reasons, over the standard visual display 130. From here said module 160 can either rotate or slide back and forth in order to expose the visual display. Of course another way would be that to plug the module 160 in and off every time this is desired. In figure 2 the tactile display module 160 is schematically represented with a cut corner in order to show the presence of the visual display 130 underneath.

When the user is not visually impaired, just the medical device 100 is preferably used. When the user is visually impaired, the medical device 100 with the module 160 plugged into it is preferably used. In this way, visually impaired users can read and operate the medical device 100 by means of the tactile display module 160. Upon need or wish, however, also users not visually impaired can read and operate the medical device 100 by means of the standard visual display 130.

## Claims

1. System comprising a medical device (100) and a tactile display module (160), wherein the medical device (100) comprises:
- an analytical test section (110) or a drug administration section (111) or both;
- a visual display (130) for users not visually impaired displaying data generated by the medical device (100) and/or giving instructions for operating the medical device (100);
wherein the tactile display module (160) is connected to, preferably plugged into the medical device; and
wherein the tactile display module (160) can transmit data generated by the medical device (100) and/or give instructions for operating the medical device (100) to visually impaired users.

2. The system of claim 1 wherein the tactile display module is a Braille module comprising a sheet display based on the Braille code or a Braille-like module comprising a sheet display for displaying contours of conventional signs, preferably language signs.

3. The system of claim 2 wherein the sheet display comprises organic field-effect transistors and plastic actuators.

4. The system according to any of the claims 1 to 3 wherein the tactile display module is connected to the medical device via a display interface.

5. The system of claim 1 wherein the visual display is an e-paper display.

6. The system according to any of the preceding claims wherein the tactile display module is disposed over the visual display .

7. Medical device (100) comprising:
- an analytical test section (110) or a drug administration section (111) or both;
- a visual display (130) for users not visually impaired displaying data generated by the medical device (100) and/or giving instructions for operating the medical device (100); and
- a display interface (140) into which a tactile display module (260) for visually impaired users transmitting data generated by the medical device (100) and/or giving instructions for operating the medical device (100) can be plugged.

8. The medical device of claim 7 wherein the visual display is an e-paper display.

9. Tactile display module for use in a system according to claims 1 to 6 wherein the display module is a Braille module comprising a sheet display based on the Braille code or a Braille-like module comprising a sheet display for displaying contours of conventional signs, preferably language signs

10. The tactile display module of claim 9 wherein the sheet display comprises organic field-effect transistors and plastic actuators.

11. Method of using the medical device of claims 7 to 8 **characterized in that** when the user needing the medical device is visually impaired a tactile display module is connected to, preferably plugged into the medical device so that both visually impaired users and users not visually impaired can read the data generated or instructions given by the medical device and/or control the medical device.

12. Use of the medical device according to claims 7 to 8 for self testing and monitoring the concentration of analytes in a body fluid, or a blood parameter, or for the controlled administration of therapeutic agents.
